# FASCICULE DE BREVET EUROPEEN

(11) **EP 2 812 014 B1**
(45) Date de publication et mention de la délivrance du brevet: **25.10.2017**
(21) Numéro de dépôt: 13701811.5
(22) Date de dépôt: 02.01.2013
(51) Int. Cl.: A61P 17/16

(54) **EXTRAIT DE GRAINES DE KNIPHOFIA UVARIA, COMPOSITION COSMETIQUE OU DERMATOLOGIQUE EN CONTENANT, ET SES UTILISATIONS**
EXTRAKT AUS KNIPHOFIA UVARIA-SAMEN, KOSMETISCHE ODER DERMATOLOGISCHE ZUSAMMENSETZUNG DAMIT UND VERWENDUNGEN DAVON
EXTRACT OF KNIPHOFIA UVARIA SEEDS, COSMETIC OR DERMATOLOGICAL COMPOSITION CONTAINING SAME, AND USES THEREOF

(30) Priorité: 04.01.2012 FR 1250077
(43) Date de publication de la demande: 17.12.2014
(73) Titulaire: LvmH Recherche, 45800 Saint-Jean De Braye (FR)
(72) Inventeur: PECHER, Virginie, F-45380 La Chapelle St Mesmin (FR); LEPLANQUAIS, Virginie, F-45450 Donnery (FR); COLIN, Anne-Sophie, F-45800 St Jean de Braye (FR); FRANCHI, Jocelyne, F-45410 Saint Jean de la Ruelle (FR); RENIMEL, Isabelle, F-45470 Trainou (FR); LAZOU, Kristell, F-45000 Orleans (FR)
(74) Mandataire: Mena, Sandra
(86) Numéro de dépôt international: PCT/FR2013/050001
(87) Numéro de publication internationale: WO 2013/102727

(56) Documents cités:
- BOROSS LASYLO: "Isolation and identification of the antibacterial substance of Kniphofia uvaria", ACTA CHIMICA ACADEMIAE SCIENTIARUM HUNGARICA, BUDAPEST, HU, vol. 35, 1 janvier 1963 (1963-01-01), pages 195-198, XP009159695, ISSN: 0001-5407
- LUCAS E H ET AL: "THE OCCURRENCE OF ANTIBACTERIAL SUBSTANCES IN SEED PLANTS WITH SPECIAL REFERENCE TO MYCOBACTERIUM TUBERCULOSIS", BULLETIN OF THE TORREY BOTANICAL CLUB, vol. 78, no. 4, 1 juillet 1951 (1951-07-01), pages 310-321, XP001100082, TORREY BOTANICAL CLUB, LAWRENCE, KS, US ISSN: 0040-9618
- Anonymous: "PARFUMS CHRISTIAN DIOR PRESS FILE APRIL 2009", , 4 février 2009 (2009-02-04), XP002676771, Extrait de l'Internet: URL:http://www.tfwa.com/duty_free/fileadmi n/user_upload/ap/090313_dior.pdf [extrait le 2012-06-06]
- DATABASE GNPD [Online] Mintel; juin 2011 (2011-06), anonymous: "Satin Revitalizing Creme", XP002676772, extrait de www.gnpd.com Database accession no. 1564943
- FERENCZY L: "Antibacterial substances in seeds", NATURE: INTERNATIONAL WEEKLY JOURNAL OF SCIENCE, NATURE PUBLISHING GROUP, UNITED KINGDOM, vol. 178, no. 4534, 22 septembre 1956 (1956-09-22), pages 639-640, XP009159752, ISSN: 0028-0836

## Description

L'invention concerne un extrait de graines de la plante *Kniphofia uvaria,* une composition cosmétique ou dermatologique comprenant ledit extrait, et son utilisation dans le traitement du vieillissement de la peau et des maladies inflammatoires cutanées.

### ETAT DE LA TECHNIQUE

La plante *Kniphofia uvaria* est une espèce rhizomateuse vivace de la famille des Liliaceae (classification classique) et des Asphodelaceae (classification phylogénétique). Cette espèce rustique possède des feuilles étroites et retombantes, pouvant atteindre un mètre de long. Les inflorescences forment un épi composé de nombreuses fleurs tubulaires aux couleurs flamboyantes.

Les fleurs produisent pendant deux mois de l'année une grande quantité de nectar, lequel contient essentiellement de l'eau, des acides aminés, du glucose, du fructose, des sels minéraux et des oligo-éléments. Ce nectar contient donc tous les éléments de base nécessaires à la nutrition cellulaire, mais il contient également de la superoxydismutase, une enzyme luttant contre la lipoperoxydation provoquée par les radicaux libres.

Compte-tenu de sa composition, le nectar des fleurs de *Kniphofia uvaria* est particulièrement adapté au soin des peaux sèches pour les nourrir en profondeur et les revitaliser, et il a déjà été incorporé dans un soin cosmétique régénérant et revitalisant. Il a également été proposé dans un autre soin cosmétique en combinaison avec des actifs anti-âge dont il complète l'action.

La demanderesse a mis en évidence de façon tout à fait surprenante qu'un extrait de graines de la plante *Kniphofia uvaria* est lui-même doté d'une activité anti-âge, si bien qu'il n'est pas nécessaire d'adjoindre à cet extrait particulier un autre actif anti-âge pour proposer un soin permettant de lutter contre le vieillissement cutané.

A ce jour, aucun procédé d'extraction mettant en oeuvre les graines de la plante *Kniphofia uvaria* n'a été décrit. Il est donc particulièrement surprenant d'avoir mis en évidence qu'un tel extrait présente une activité cosmétique.

Les inventeurs de la présente invention ont ainsi tout d'abord mis en évidence qu'un extrait de graines de la plante *Kniphofia uvaria,* en particulier extrait lipophile de ces graines, diminuent la production de prox-Matrix MetalloProtéinase de type 1 (pro-MMP1), enzyme clé responsable de la dégradation de la matrice extracellulaire, laquelle dégradation est particulièrement accélérée par les rayonnements UV.

Il a été également montré qu'un traitement in vitro de fibroblastes par l'extrait de l'invention provoque une forte inhibition de l'expression des métalloprotéinases, plus particulièrement les MMP-2 et MMP-9, enzymes responsables de la dégradation des protéines de la matrice extracellulaire.

Il a aussi été démontré qu'un extrait de l'invention est un agoniste des récepteurs activés par les proliférateurs de peroxisomes (PPARs) dont un isotype β/δ est prépondérant dans l'épiderme. Cette interaction entre l'agoniste et le récepteur PPAR provoque l'activation de la transcription de gènes cibles, notamment impliqués dans la différenciation des kératinocytes. Ce processus conduit ainsi à la formation du stratum corneum, notamment en stimulant la synthèse de lipides épidermiques, en augmentant la formation et la sécrétion des corps lamellaires ou encore en augmentant l'activité d'enzymes impliquées dans le mécanisme de sécrétion extra cellulaire des lipides au niveau de la couche cornée (Schmuth M. et al., J. Lipids Res., 2008, 49, 499-509).

Les PPARs sont aussi connus pour leur activité anti-inflammatoire (Man M. et al 2008, J. Invest. Dermatol., 128, 370-377), en ciblant notamment les interactions neutrophiles-cellules endothéliales (Piqueras L. et al 2009, J. Leucocyte Biol., 86, 115-122).

Par ailleurs, le statut micro inflammatoire (ou la notion d'inflammation stérile) des tissus âgés est de plus en plus reconnue, le phénotype pro-inflammatoire des cellules sénescentes perturbant l'homéostasie tissulaire. Les MMPs font parties des facteurs sécrétés par les cellules sénescentes comme source d'inflammation chronique dommageable pour les cellules environnantes et le tissu (Freund A. et al, 2010, Trends Mol. Med., 16, 238-246).

Enfin, il a été montré par les inventeurs que cet extrait possède la propriété de moduler l'expression de gènes codant pour certaines protéines impliquées dans plusieurs processus biologiques liés au vieillissement de la peau, à son état d'hydratation de la peau et des tissus, ou encore à son niveau de fermeté et/ou de son élasticité.

De par son activité vis-à-vis de ces cibles, un tel extrait présente un intérêt pour une utilisation en tant qu'agent actif dans des compositions cosmétiques ou dermatologiques, notamment destinées à lutter contre le vieillissement cutané, à contribuer au maintien de la fermeté, et/ou à maintenir la fonction barrière de la peau.

Par ailleurs, lorsque l'extrait selon l'invention a une consistance huileuse, il est particulièrement intéressant de l'utiliser comme excipient cosmétique, plus particulièrement comme agent texturant de phases grasses de compositions cosmétiques.

### BUTS DE L'INVENTION

L'invention a pour but principal de fournir un nouvel extrait d'origine végétale, notamment utilisable en tant qu'agent actif et/ou excipient dans des compositions cosmétiques ou dermatologiques, notamment destinées à prévenir ou retarder l'apparition des signes du vieillissement cutané ou à en ralentir ou atténuer les effets.

L'invention a aussi pour but de fournir un nouvel extrait d'origine végétale pour lutter contre les effets induits par l'exposition des cellules de la peau aux UV, tels que la dégradation de la matrice extracellulaire et/ou l'installation d'un statut micro inflammatoire délétère à terme, ou bien encore de contribuer au maintien de la fermeté et/ou de la fonction barrière de la peau.

L'invention a également pour but de fournir une composition cosmétique contenant ce nouvel extrait, ainsi qu'une méthode de soin cosmétique utilisant cette composition. L'invention propose également une composition dermatologique contenant ce nouvel extrait.

L'invention a enfin pour but de résoudre l'ensemble des problèmes techniques par une solution simple, relativement peu coûteuse et utilisable à l'échelle industrielle, en particulier dans l'industrie cosmétique.

### DESCRIPTION DE L'INVENTION

L'invention est telle que revendiquée.

Ainsi, selon un premier objet, l'invention concerne un extrait des graines de la plante *Kniphofia uvaria,* Cet extrait est obtenu par mise en contact desdites graines avec du dioxyde de carbone à l'état supercritique comme solvant, ou bien par pressage mécanique à froid desdites graines.

L'extrait de graines est de préférence un extrait lipophile. Par «extrait lipophile », on entend un extrait obtenu par mise en contact des graines avec un solvant apolaire ou par pressage mécanique des graines. Selon une mise en oeuvre préférée, l'extrait de graines est une huile liquide à température ambiante (25°C).

Préalablement à l'étape d'extraction par le dioxyde de carbone supercritique ou préalablement au pressage mécanique, les graines récoltées peuvent avoir été séchées et/ou broyées.

L'invention a pour objet un extrait végétal lipophile de la plante *Kniphofia uvaria* obtenu par un procédé d'extraction dans lequel le solvant d'extraction est le dioxyde de carbone à l'état supercritique. La partie de la plante utilisée est de préférence les graines.

On définit l'état supercritique d'un fluide, comme étant l'état dans lequel se trouve ce fluide, quand on le soumet à des conditions de température et de pression telles que la température appliquée est supérieure à une température critique (Tc) et la pression appliquée est supérieure à une pression critique (Pc), ces valeurs critiques étant spécifiques à chaque fluide.

Pour le dioxyde de carbone, la température critique Tc est égale à 31°C et la pression critique Pc est égale à 7,38.10⁶ Pa.

Selon un mode de réalisation, le dioxyde de carbone est à l'état supercritique à une température allant de 35°C à 80°C, et une pression supérieure à 7,4.10⁶ Pa.

On définit l'état subcritique comme l'état dans lequel se trouve un fluide, quand la température à laquelle il est soumis est inférieure à la température critique Tc (Tc=31°C pour le dioxyde de carbone), la pression pouvant être alors indifféremment inférieure ou supérieure à la pression critique (Pc=7,38.10⁶ Pa pour le dioxyde de carbone).

Les conditions de température et de pression sont adaptées de façon à placer le dioxyde de carbone dans l'état souhaité, cependant la pression peut représenter une valeur allant jusqu'à 300 fois la pression atmosphérique (1 atm = 0,101.10⁶ Pa), mais on utilise préférentiellement une pression comprise entre 8.10⁶ Pa et 30.10⁶ Pa pour une extraction au dioxyde de carbone à l'état supercritique, et comprise entre 6,5.10⁶ Pa et 10.10⁶ Pa, pour une extraction à l'état subcritique.

Avantageusement, le dioxyde de carbone est comprimé à une pression supérieure à 7,4.10⁶ Pa, de préférence supérieure ou égale à 18.10⁶ Pa et de manière particulièrement préférée supérieure ou égale à 25.10⁶ Pa.

Avantageusement encore, l'extraction est effectuée à une température comprise entre 35°C et 80°C.

A titre de moyens optionnels et/ou additionnels dans un tel procédé d'extraction, on peut utiliser des solvants organiques en tant que co-solvant ou agent d'entraînement, pour modifier la polarité du mélange formé avec le dioxyde de carbone, renforcer le pouvoir solvant vis-à-vis de certaines molécules peu ou pas solubles dans le dioxyde de carbone à l'état supercritique et/ou pour faciliter l'entraînement du mélange formé.

On cite à titre d'exemple l'éthanol comme co-solvant utilisable pour modifier la polarité du mélange formé avec le dioxyde de carbone, ou bien des esters d'acides gras tels que par exemple, le dicaprylyl carbonate (Cetiol CC®, Cognis GmbH), le cétéaryl isononanoate Cetiol SN®, Cognis GmbH) ou encore le caprylic/capric triglycéride (Mygliol 812®, Hüls AG), utilisable en tant qu'agent d'entraînement.

Quand on utilise l'un de ces solvants au cours du procédé d'extraction, sa concentration est avantageusement inférieure ou égale à 5% en poids par rapport au poids de dioxyde de carbone utilisé pour l'extraction.

A la fin de l'étape d'extraction proprement dite, une phase de détente par abaissement de la pression et éventuellement de la température, provoque le passage du dioxyde de carbone de l'état supercritique à l'état gazeux, ce qui permet d'éliminer complètement le dioxyde de carbone de l'extrait obtenu. On élimine également au cours de cette étape le solvant organique optionnellement utilisé à titre d'agent d'entraînement ou de co-solvant.

Le procédé d'extraction peut en outre être complété par une étape d'élimination partielle ou totale des solvants d'extraction.

Cette étape de séchage consiste préférentiellement en une opération de lyophilisation de l'extrait obtenu ou une étape de chauffage, avantageusement sous vide.

Afin d'améliorer l'aspect organoleptique de l'extrait, le procédé d'obtention de l'extrait peut en outre comprendre avantageusement une étape de désodorisation et/ou de décoloration, par entraînement à la vapeur, par distillation moléculaire ou décoloration au charbon actif.

L'extrait de l'invention est obtenu par mise en contact de graines de la plante *Kniphofia uvaria* avec le dioxyde de carbone à l'état supercritique, en absence de co-solvant.

Selon un mode préféré, on utilise des graines préalablement séchées.

Selon une mise en oeuvre, l'extraction est effectuée à une température allant de 35°C à 80°C et de manière particulièrement préférée de 60°C. Avantageusement, l'extraction des graines de la plante est réalisée à la température de 60°C et à la pression de 290 bars (29.10⁶ Pa).

Selon une alternative au procédé d'extraction décrit précédemment, on peut obtenir l'extrait de l'invention en soumettant les graines de la plante à un pressage mécanique. Ce pressage est réalisé à froid, de préférence sur les graines non broyées, qui peuvent avoir été préalablement séchées.

Un autre objet de l'invention concerne l'utilisation d'un extrait végétal des graines de la plante *Kniphofia uvaria,* de préférence un extrait huileux des graines, en tant qu'agent actif et/ou excipient dans une composition cosmétique.

Cet extrait est particulièrement adapté à un usage cosmétique, notamment dans des compositions cosmétiques comprenant au moins une phase grasse. Sa consistance le rend d'un grand intérêt pour une utilisation en tant qu'excipient dans des compositions cosmétiques, en particulier dans celles comprenant au moins une phase grasse ou constitués d'une phase grasse.

Selon un deuxième objet, l'invention concerne une composition cosmétique ou dermatologique comprenant l'extrait décrit précédemment et au moins un excipient cosmétiquement ou dermatologiquement acceptable.

La composition comprend de préférence une quantité efficace de l'extrait pour obtenir l'effet recherché. La composition comprend ainsi préférentiellement de 0,0001% à 10% en poids sec d'extrait, de préférence de 0,01% à 5% en poids, par rapport au poids total de la composition. Les pourcentages en poids sec sont exprimés sur la base du poids de l'extrait ne comprenant pas, ou comprenant à l'état de traces du solvant d'extraction.

A titre d'exemple, l'extrait végétal lipophile peut être contenu dans la phase grasse d'émulsions huile-dans-eau telles que des crèmes de soin de la peau, ou dans des phases grasses de compositions de maquillage telles que des rouges à lèvres ou des mascaras.

Ces compositions peuvent comprendre une phase aqueuse ou bien être sensiblement anhydres, c'est-à-dire ne comporter que des traces d'eau résiduelle.

L'extrait de l'invention peut être également associé dans des compositions cosmétiques ou dermatologiques, avec d'autres agents actifs cosmétiquement acceptables, sous forme de molécules purifiées et/ou d'extraits, notamment d'extraits végétaux, présentant des effets cosmétiques similaires et/ou complémentaires de ceux dudit extrait de l'invention.

De tels agents actifs peuvent être choisis parmi les substances ayant une activité éclaircissante de la peau; les substances ayant une activité amincissante; les substances ayant une activité hydratante ; les substances ayant une activité calmante, apaisante ou relaxante; les substances ayant une activité

A titre d'exemple, l'extrait végétal lipophile peut être contenu dans la phase grasse d'émulsions huile-dans-eau telles que des crèmes de soin de la peau, ou dans des phases grasses de compositions de maquillage telles que des rouges à lèvres ou des mascaras.

Ces compositions peuvent comprendre une phase aqueuse ou bien être sensiblement anhydres, c'est-à-dire ne comporter que des traces d'eau résiduelle.

L'extrait de l'invention peut être également associé dans des compositions cosmétiques ou dermatologiques, avec d'autres agents actifs cosmétiquement acceptables, sous forme de molécules purifiées et/ou d'extraits, notamment d'extraits végétaux, présentant des effets cosmétiques similaires et/ou complémentaires de ceux dudit extrait de l'invention.

De tels agents actifs peuvent être choisis parmi les substances ayant une activité éclaircissante de la peau; les substances ayant une activité amincissante; les substances ayant une activité hydratante ; les substances ayant une activité calmante, apaisante ou relaxante; les substances ayant une activité stimulant la microcirculation cutanée pour améliorer l'éclat du teint, en particulier du visage; les substances ayant une activité sébo-régulatrice pour le soin des peaux grasses; les substances destinées à nettoyer ou purifier la peau; les substances ayant une activité anti-radicalaire; les substances destinées à atténuer ou retarder les effets du vieillissement de la peau, en particulier la formation de rides, par une activité visant à favoriser le maintien de la structure de la peau et/ou à limiter la dégradation de la matrice extracellulaire des couches superficielles du derme et de l'épiderme et/ou à obtenir un effet protecteur, correcteur ou restructurant de la peau; les substances ayant une activité anti-inflammatoire.

Avantageusement, la composition de l'invention comprend en outre au moins un excipient cosmétiquement ou dermatologiquement acceptable pouvant être choisi parmi des pigments, des colorants, des polymères, des agents tensioactifs, des agents de rhéologie, des parfums, des électrolytes, des ajusteurs de pH, des agents anti-oxydants, des conservateurs, et l'un quelconque de leurs mélanges.

La composition peut être par exemple un sérum, une lotion, une crème, une émulsion huile-dans-eau, un hydrogel, un masque, une émulsion huile-dans-eau, ou encore se présenter sous la forme d'un stick, d'un patch, ou un produit de maquillage de type rouge-à-lèvres, mascara ou fond de teint.

L'extrait peut encore être utilisé dans une composition dermatologique:
- pour préserver la matrice extracellulaire d'une dégradation, notamment accélérée par les rayonnements UV,
- pour limiter le processus d'inflammation chronique dommageable pour la peau liée aux sécrétions des cellules stressées par les radicaux libres endogènes ou produits par les UV et/ou sénescentes,
- pour apaiser des peaux irritées par exemple sous l'effet des UV ou de l'utilisation d'alpha-hydroxyacides, notamment lors d'un gommage ou d'un « peeling ».

Un quatrième objet de l'invention vise enfin une méthode de soin cosmétique comprenant l'application sur au moins une partie de la peau du visage ou du corps présentant des signes du vieillissement - comme des rides ou un relâchement- d'une quantité efficace d'une composition ou d'un extrait tels que définis précédemment, pour traiter les signes du vieillissement intrinsèque ou extrinsèque de la peau, ou pour traiter les problèmes de sécheresse cutanée liée au vieillissement qui mettent en jeu une baisse de la production de lipides qui contribue à la perturbation de la fonction barrière de la peau.

L'invention a encore pour objet une composition dermatologique pour son utilisation dans le traitement du processus d'inflammation chronique dommageable pour la peau liée aux sécrétions des cellules stressées et/ou sénescentes, ou dans le traitement de la réactivité cutanée des peaux sensibles et/ou l'irritation de la peau d'un patient.

L'invention concerne aussi une composition dermatologique telle que décrite précédemment pour son utilisation dans le traitement des signes du vieillissement intrinsèque ou extrinsèque de la peau, et/ou dans le traitement du processus d'inflammation chronique dommageable pour la peau liée aux sécrétions des cellules stressées et/ou sénescentes.

Avantageusement, la composition dermatologique est appliquée sur une zone de peau du corps ou du visage présentant des signes visibles de vieillissement tels que la présence de rides ou de ridules, une irritation, des signes inflammatoires, une perte d'éclat du teint, une perte de fermeté et/ou d'élasticité de la peau ou d'autres signes tels qu'une diminution de l'épaisseur de la peau, une augmentation de la sécheresse ou de la rugosité cutanée.

Avantageusement, la composition cosmétique est appliquée sur une zone de peau du corps ou du visage présentant des signes perceptibles d'inconfort ou de sensibilité.

D'autres buts, caractéristiques et avantages de l'invention apparaîtront clairement à la lumière de la description explicative qui va suivre faite en référence à des exemples de préparation d'extrait et de tests mettant en évidence les propriétés de l'extrait et à des exemples de composition cosmétique utilisant un tel extrait, qui sont donnés à titre d'illustration de l'invention sans pour autant en limiter la portée.

Dans les exemples, tous les pourcentages sont donnés en poids, la température est en degrés Celsius, la pression est la pression atmosphérique, sauf indication contraire.

### EXEMPLES

### Exemple 1 : Préparation d'un extrait de graines de Kniphofia uvaria.

### Préparation d'extraits de l'invention à l'aide d'un solvant apolaire.

Les graines, collectées sur les plantes de l'espèce *Kniphofia uvaria* ont été séchées puis broyées.

La matière végétale résultant de ce broyage a été extraite à l'aide de CO₂ à l'état supercritique, en absence de co-solvant.

Les conditions du procédé d'extraction étaient les suivantes :
Pression = 29 10⁶ Pa (290 bars)/Température = 60°C (dans ces conditions de pression et de température, le dioxyde de carbone est à l'état supercritique).

On a séché l'extrait obtenu par distillation à l'évaporateur rotatif, sous vide, à 50°C, en présence d'éthanol (5% en poids par rapport à l'extrait).

L'extrait obtenu (EXTRAIT 1) après séchage était une huile limpide de couleur orange. Le rendement de l'extraction était de 25% en poids par rapport au matériel végétal mis en oeuvre.

### Préparation d'extraits de l'invention par pressage à froid.

Les graines entières et préalablement séchées sont pressées mécaniquement à froid, à l'aide d'une presse adaptée à cet usage.

L'huile collectée est filtrée.

On la soumet à une étape de désodorisation à la vapeur d'eau, pour en améliorer les propriétés organoleptiques.

Dans les exemples ci-après, le terme « extrait » utilisé seul fait référence à l'extrait de l'invention préparé conformément à l'exemple présent par l'un ou l'autre des moyens décrits.

### Exemple 2 - Tests d'activité in vitro d'un extrait de l'invention

### 1) Marqueurs MMP-2, MMP- 9, pro MMP-1

### 1.1) Activités biologiques

Préalablement au traitement, on a préparé une solution-mère de l'extrait de l'exemple 1 solubilisé dans du DMSO à la concentration de 6,25 et 12,5 mg d'extrait par mL de solvant, ou des témoins.

Au moment du traitement des cellules par l'extrait de l'invention, la solution-mère est diluée au 1/1000^{ème} dans le milieu de culture pour atteindre la concentration souhaitée.

Un extrait *d*'*Anogeissus Leiocarpus* et le resvératrol, dont on connaît la capacité à inhiber la synthèse de ces protéines par les fibroblastes en culture, sont utilisés en tant que témoins positifs.

Les tests sont menés sur fibroblastes humains normaux (FHN).

Les FHN sont ensemencés à la densité de 5000 cellules/puits et 200 µL/puits de milieu de culture MEM (Gibco Invitrogen) complété avec de la glutamine (Gibco Invitrogen, concentration finale 2 mM) et 10% de SVF (sérum de veau foetal), dans une microplaque 96 puits (Falcon). La plaque de culture est placée à l'étuve pendant 24 heures afin d'obtenir 80% de confluence cellulaire.

On traite les cellules avec d*'Anogeissus Leiocarpus* à 25 µg/mL et un témoin solvant (DMSO). On prépare également un témoin UV-B en soumettant les fibroblastes à des UV-B, de façon à valider la stimulation de la sécrétion de MMPs par les cellules sous l'influence de ce facteur.

L'extrait à différentes concentrations, le témoin positif et le témoin solvant, sont chacun évalués sur 4 puits de FHN.

Après 48 heures de traitement, les surnageants de culture sont prélevés et stockés à -20°C. On utilise la technique immunoenzymatique du «sandwich», qui permet de doser dans les surnageants, par mesure spectrophotométrique à 450 nm, les quantités respectives de MMP-2, MMP-9 et pro-MMP-1. Les dosages de MMP-2, 9 et pro MMP-1 s'effectuent selon les protocoles décrits dans les kits Quantikine (R&D Systems).

### 1.2) Résultats

Les résultats sont présentés dans les tableaux ci-dessous

**Tableau 1 : Dosage MMP-9**

| | T DMSO | T+UVB | T+ | EXTRAIT 1 | EXTRAIT 1 |
|---|---|---|---|---|---|
| Dose (µg/mL) | | | | 6,25 | 12,5 |
| moyenne (ng MMP-9/µg prot) | 0.0202 | 0.0539 | 0.0251 | 0.0449 | 0.0369 |
| écartype | 0.0005 | 0.0017 | 0.0010 | 0.0046 | 0.0003 |
| % inhibition | | | *53.46* | *16.73* | *31.52* |

### Légendes du tableau :

*T DMSO = témoin solvant de solubilisation, (DMSO)*,
*T+UVB = témoin positif de stimulation aux UVB produisant une augmentation du taux de MMP-9,*
*T+ = témoin positif du test (anogelline),*

**Tableau 2 : Dosage MMP-2**

| | T DMSO | T+ | 12,5 µg/mL | 6,25 µg/mL |
|---|---|---|---|---|
| moyenne (ng MMP-2/µg prot) | 0.1437 | 0.0416 | 0.1281 | 0.1215 |
| % inhibition | | 71.07 | 10.86 | 15.44 |

**Tableau 3 : Dosage pro MMP-1**

| | T DMSO | T+UVB | T+ | 12,5 µg/mL |
|---|---|---|---|---|
| moy (ng MMP1/µg prot) | 0.0196 | 0.0291 | 0.0137 | 0.0017 |
| % inhibition | | | 52.8 | 94.1 |

### 1.2) Conclusions

MMP-2 est exprimée par les fibroblastes pendant le développement et la régénération tissulaire. Avec la MMP-9, cette protéine dégrade le collagène type IV, composé majeur des membranes basales et de la gélatine (collagène dénaturé). Elle peut aussi dégrader d'autres types de collagènes (V, VII et X) tels l'élastine et la fibronectine. Les substrats de MMP-9 peuvent être des collagènes natifs type IV, V, VII, X et XI, la fibronectine. La MMP-1 est une enzyme majeure impliquée dans la dégradation de la matrice extracellulaire. A la différence de MMP-2, la production de MMP-1 est gouvernée par le facteur de transcription AP-1 qui est directement influencé par des stress environnementaux, comme les rayonnements UV, qui accélèrent le vieillissement cutané.

Le traitement des cellules à l'aide d'une solution de l'extrait de graines de *Kniphofia uvaria* a permis d'inhiber significativement la sécrétion de métalloprotéinases et de la Pro-MMP1.

Du fait de cette activité de l'extrait de l'invention, il est intéressant de l'utiliser dans des compositions cosmétiques pour limiter les effets du vieillissement cutané, notamment provoqué ou accéléré par les rayons UV, et contribuer ainsi à limiter le processus d'inflammation chronique dommageable pour le tissu liée aux sécrétions des cellules stressées et/ou sénescentes.

### 2) Ligand de PPAR

### 2.1) Protocole

L'essai a été réalisé sur une lignée cellulaire transfectée stable (Seimandi et al., Analytical Biochemistry, 2005, 344, 8-15) possédant un système rapporteur PPARβ/δ, exprimant une protéine chimérique contenant le domaine de liaison au ligand du PPARβ/δ humain fusionné avec le domaine de liaison à l'ADN du facteur de transcription de levure GAL-4 (DBD).

Le gène rapporteur de la luciférase était sous le contrôle d'un pentamère de la séquence de reconnaissance GAL-4 situé devant le promoteur de la β-globuline. *(*Normand et al., Proceedings MipTec - The 9th International Conference and Exhibition on Drug Discovery, 08.05.2006 - 11.05.2006, P 105).

Les composés ont été testés en plaques 96 puits dans lesquels ont été ensemencées les cellules. Celles-ci ont été incubées pendant 24 heures avec l'actif testé. Les cellules ont été amenées à confluence supérieure à 80%. L'extrait de l'exemple 1 a été dissous dans le DMSO puis dilué dans le milieu de culture pour atteindre la concentration recherchée. On a mesuré l'activité luciférase par luminescence. La sélectivité de composés pour les récepteurs PPAR est déterminée par comparaison avec une référence et d'un contrôle négatif (0,1% DMSO).

Pour déterminer l'activité de l'extrait de l'invention sur les sous-types de récepteurs PPAR, on a suivi l'activité de la luciférase en luminescence correspondant à l'activation du PPARβ/δ. Les valeurs obtenues ont été exprimées en % d'activité luciférase du ligand testé comparativement à l'activité d'un agoniste pharmacologique de référence fixée à 100% (L16541 1 µM, TEBU-BIO).

### 2.2) Résultats

Pour l'extrait de *Kniphofia uvaria* de l'exemple 1, le test a donné les valeurs d'activité ci-dessous :
- à 10 µg/mL dans le milieu de culture : la réponse représente 14% de l'effet du témoin positif (100% agoniste).
- à 30 µg/mL dans le milieu de culture : la réponse représente 37% de l'effet du témoin positif.
- à 50 µg/mL dans le milieu de culture : la réponse représente 43 % de l'effet du témoin positif (100% agoniste).

### 2.3) Conclusions

Les récepteurs activés par les proliférateurs de peroxisomes (PPARs) sont des facteurs de transcription qui appartiennent à la superfamille des récepteurs nucléaires. Les PPARs se fixent sur une région spécifique de l'ADN située dans la région régulatrice de gènes cibles, principalement impliqués dans le métabolisme des lipides. Le PPARβ/δ représente l'isotype prépondérant dans l'épiderme humain, avec un fort taux au niveau des kératinocytes (Girroir et al., Biochem. Biophys. Res. Comm., 2008, 371, 456-461).

Comme expliqué dans la partie introductive, les PPARs exercent un rôle capital dans la physiologie de l'épiderme, en particulier vis-à-vis de sa qualité de fonction barrière, et de son rôle régulateur du statut inflammatoire et qu'il est ainsi très intéressant d'identifier des agonistes de ces récepteurs.

L'huile de Kniphofia est un agoniste d'origine végétale puissant des PPARβ/δ, avec un effet dose-dépendant.

Cette activité vis-à-vis des récepteurs PPAR rend cet extrait utilisable en cosmétique dans des applications visant à traiter des désordres cutanés, en particulier les problèmes de sécheresse cutanée, éventuellement liée au vieillissement, qui mettent en jeu une baisse de la production de lipides qui contribue à la perturbation de la fonction barrière de la peau *(*Ghadially R. et al, J. Clin. Invest., 1995, 95, 2281-2290*,* Seyfarth F. et al., Clinics Dermatol., 2011, 29, 31-36).

Le potentiel anti-inflammatoire de cet extrait, agoniste de PPARβ/δ, permet d'envisager en outre son utilisation en tant qu'agent actif, notamment pour moduler la réactivité cutanée des peaux sensibles, pour l'apaisement des peaux irritées par exemple sous l'effet des UV, ou encore pour limiter le cycle dommageable de micro-inflammation chronique qu'initient les cellules sénescentes dans les peaux âgées.

### Exemple 3 -Effet du traitement de fïbroblastes humains normaux par l'extrait de l'invention sur l'expression de gènes.

Le but de cette étude était d'étudier l'activité biologique d'un extrait de l'invention, sur l'expression de gènes codant pour des protéines impliquées dans des processus biologiques liés au vieillissement cutané ou l'état d'hydratation de la peau sur une culture de fibroblastes humains normaux (FHN) cultivés en monocouche.

La technologie TLDA pour Taqman Low density Array, permet l'étude de la modulation de l'expression d'un panel de gènes, codant pour des protéines spécifiques de voies biologiques liées aux fibroblastes, en réponse à un traitement d'une durée de 24h par l'extrait obtenu à l'exemple 1 (EXTRAIT 1).

### 1. Matériels et Méthodes

### Culture cellulaire

Les fibroblastes humains normaux (FHN), issus d'un donneur adulte caucasien, ont été ensemencés en plaques 6 puits à raison de 2.5.10⁴ cellules/puits dans du milieu 1 de composition ci-dessous :

| *Milieu 1* | | |
|---|---|---|
| | *Fournisseur* | *Concentration finale* |
| *SVF* | *Biowest* | *10%* |
| *DMEM* | *Fisher* | *qs* |

Trois puits de FHN ont été ensemencés par condition de culture.

24 heures avant le traitement, à confluence, les cellules ont été déplétées en sérum de veau foetal (milieu 2) de composition ci-dessous :

| *Milieu 2* | |
|---|---|
| | *Fournisseur* |
| *DMEM* | *Fisher* |

### 2. Traitement

Après 24 heures de culture sans sérum de veau foetal, les cellules ont été traitées par l'EXTRAIT 1 de l'invention, à 0,1% en poids dans du milieu 2. Après 24 heures de traitement, les cellules ont été récupérées afin d'en extraire les ARN totaux.

Un témoin non traité est également réalisé dans les mêmes conditions.

### 3. PCR Taqman Low Density Array

### 3.1 Obtention des ARN totaux

Le milieu de culture des cellules a été éliminé, et 250 µL de tampon de lyse RLT (fourni dans le kit Nucleospin RNA trace) ont été ajoutés. Les cellules ont été raclées à l'aide d'un *Cell Scraper* puis le lysat cellulaire, récupéré dans une deepwell 1,2 mL (fourni dans le kit). Les ARN totaux ont été extraits à l'aide d'un Epimotion 5075 (Eppendorf) avec le kit Nucleospin RNA trace (Macherey Nagel).

Les solutions d'ARN totaux obtenues ont été dosées et leur qualité vérifiée, à l'aide du Bioanalyseur 2100 (Agilent Technologies). Cet appareil était relié à un ordinateur possédant le logiciel spécifique d'analyse des résultats (logiciel 2100 expert). La technique a nécessité une micro-plaque de 12 puits (RNA 6000 NanoChips) et un kit de réactifs (RNA 6000 Nano Reagents & Supplies), spécifiques du dosage des ARN totaux eucaryotes

### 3.2 Synthèse des ADN complémentaires

Le kit de reverse transcription (RT) utilisé était le High Capacity cDNA Reverse Transcription Kit (APPLIED IOSYSTEMS). 100 ng d'ARN totaux, ont été dilués dans de l'eau pour un volume final de 50 µL. Ils ont été ensuite incubés pendant 10 minutes à 25°C puis 2 heures à 37°C avec 50 µL de mélange réactionnel de High capacity reverse transcription kit 2X préalablement préparé comme indiqué ci dessous.

| *Réactifs* | *volume* |
|---|---|
| *RT buffer* | *10 µL* |
| *dNTP buffer* | *4 µL* |
| *Random primer* | *10 µL* |
| *RNAse out* | *1 µL* |
| *RT* | *5 µL* |
| *H₂O* | *20 µL* |

### 3.3 PCR Taqman Low Density Array

50 µL de chaque RT ont été prélevés et mélangés à 50 µL de « Taqman Gene Expression master mix ». Après homogénéisation, les 100 µL ont été déposés sur les cartes microfluidiques, ces dernières ont été centrifugées puis scellées.

On a utilisé des gènes contrôles pour la normalisation des résultats. La PCR ont été réalisée selon le protocole fourni par Applied Biosystems dans l'appareil ABI Prism 7900HT Sequence détection system. Les étapes de la qPCR ont été 2 min à 50°C, 10 min à 94,5°C puis 30 s à 97°C et 1 min à 59,7°C pour 40 cycles.

### 3.4 Analyse des résultats

Dans la méthode de RT-PCR TLDA, la quantification est effectuée en utilisant la méthode comparative de ΔCt. Cette méthode détermine le nombre de cyles (Ct) de chaque gène de la carte en utilisant le logiciel RQ Manager qui prend en compte du bruit de fond pour chaque gène. Ce nombre de cycles (Ct) ont été normalisé par rapport au Ct d'un gène de ménage GAPDH invariant dans les cellules.

### 4. Résultats

L'analyse menée sur différents gènes impliqués dans des processus liés au vieillissement cutanée, à l'hydratation de la peau ou au maintien de son élasticité et de sa fermeté ont été étudiés par la méthode décrite ci-dessous.

### Métalloprotéinase 1 (MMP-1)

La métalloprotéinase 1 (MMP-1) agit dans la dégradation des collagènes et plus particulièrement des collagènes 1 et 3. Elle est particulièrement induite au cours du vieillissement cutané et en réponse aux expositions UV. Il est donc stratégique de vouloir inhiber l'expression de cette protéine, de façon à contrôler la dégradation de la matrice extracellulaire dermique. Les résultats obtenus mettent en évidence que le traitement des FHN par l'extrait de l'invention permet de diminuer significativement (- 84%) l'expression de la MMP-1 après 24 heures de traitement, par rapport au témoin.

L'effet de l'extrait de l'invention sur l'expression de cette métalloprotéinase est particulièrement intéressant pour prévenir ou ralentir le vieillissement cutané, en limitant la dégradation de la MEC.

### Low density protein receptor related protein-1 (LRP-1).

Le traitement des FHN par l'extrait de l'invention permet d'augmenter significativement (+190%) l'expression de la protéine codée par le gène LRP-1 par rapport au témoin.

Ce gène LRP-1 code pour un récepteur au LDL, décrit pour avoir une action dans l'endocytose" des MMPs en excès dans l'espace péricellulaire.

L'effet de l'extrait de l'invention sur l'expression de ce gène est donc particulièrement intéressant pour prévenir ou ralentir le vieillissement cutané, en agissant positivement sur la dégradation de la MEC.

### Elastine (ELN)

Le traitement des FHN par l'extrait de l'invention permet d'augmenter significativement (+135%) l'expression de la tropoélastine codée par le gène ELN par rapport au témoin.

L'effet de l'extrait de l'invention sur l'expression de ce gène est donc particulièrement intéressant pour prévenir ou ralentir le vieillissement cutané, en agissant positivement sur la synthèse d'élastine.

### Collagène 1A1

Le traitement par l'extrait de l'invention permet d'augmenter significativement (+103%) l'expression du collagène 1A1 par stimulation du gène COL A1A.

L'effet de l'extrait de l'invention sur l'expression de ce gène est donc particulièrement intéressant pour prévenir ou ralentir le vieillissement cutané, en agissant positivement sur la synthèse de collagène au niveau de la matrice extracellulaire.

### Exemple 4 -Effet du traitement de kératinocytes humains normaux par l'extrait de l'invention sur l'expression de gènes.

L'objectif de cette étude a été d'évaluer l'effet d'un extrait de l'invention, sur l'expression de gènes codant pour des protéines impliquées dans des processus liés au vieillissement ou à l'hydratation de la peau.

### 1. Culture cellulaire

Les kératinocytes humains normaux (KHN) issus d'un donneur adulte caucasien ont été ensemencées en plaques 6 puits à raison de 2.5.10⁴ cellules/puits dans du milieu 1. Trois puits de KHN ont été ensemencés par condition de culture et ceci pour un traitement de 8 heures.

### 2. Traitement

A 80 % de confluence, les cellules ont été traitées avec l'extrait de l'invention de l'exemple 1, à 0,1% en poids dans du milieu 1 ci-dessous :

| | *Fournisseur* |
|---|---|
| Epilife | Fisher |
| Compléments | Fisher |

Après 24 heures de traitement, les cellules ont été récupérées afin d'en extraire les ARN totaux.

### 3. PCR Taqman Low Density Array

Le protocole est identique à celui décrit à l'exemple précédent.

### 4. Résultats

Comme à l'exemple précédent, l"analyse est menée sur des gènes impliqués dans l'expression de protéines impliquées dans des processus liés au vieillissement cutanée, à l'hydratation de la peau ou au maintien de son élasticité et de sa fermeté.

### TIMP-2

Les résultats obtenus mettent en évidence que le traitement des KHN par l'extrait de l'invention permet d'augmenter significativement (+ 30%) l'expression des protéines inhibitrices de la metalloprotéinase de type 2 après 24 heures de traitement par rapport au témoin.

Cette protéine inhibe essentiellement les métalloprotéinases MMP-2 et MMP-9. L'effet de l'extrait de l'invention sur l'expression de cette métalloprotéinase est particulièrement intéressant pour prévenir ou ralentir le vieillissement cutané, en limitant la dégradation des fibres élastiques de la Matrice Extracellulaire (MEC).

### Collagène 4A1

Les résultats obtenus mettent en évidence que le traitement des KHN par l'extrait de l'invention permet d'augmenter significativement (+ 39%) l'expression du collagène de type 4. Ce collagène joue un rôle fondamental dans le maintien de l'intégrité de la jonction dermo-épidermique.

L'effet de l'extrait de l'invention sur l'expression de ce collagène est particulièrement intéressant pour maintenir l'intégrité de la Matrice Extracellulaire (MEC) et ainsi lutter contre les effets du vieillissement de la peau.

### Aquaporine-3

Les résultats obtenus mettent en évidence que le traitement des KHN par l'extrait de l'invention permet d'augmenter significativement (+ 160%) l'expression de l'aquaporine de type 3. Ce collagène joue un rôle fondamental dans le maintien de l'intégrité de la jonction dermo-épidermique.

L'effet de l'extrait de l'invention sur l'expression de cette aquaporine au niveau des kératinocytes permet de favoriser les transferts d'eau cellulaire et améliore l'état d'hydratation des couches superficielles de la peau.

### Hyaluronan synthase-3

Les résultats obtenus mettent en évidence que le traitement des KHN par l'extrait de l'invention permet d'augmenter significativement (+ 240%) l'expression de la protéine codée par le gène HAS3. Cette enzyme est impliquée dans la synthèse de l'acide hyaluronique, qui joue un rôle important dans le maintien de l'état d'hydratation des cellules cutanées.

### CD-44

Les résultats obtenus mettent en évidence que le traitement des KHN par l'extrait de l'invention permet d'augmenter significativement (+45%) l'expression de ce récepteur de l'acide hyaluronique. Cet effet significatif permet d'améliorer ou de maintenir l'état d'hydratation des cellules cutanées traitées par un extrait de l'invention.

### Exemple 5 - Crème pour le visage

L'extrait de graines de *Kniphofia uvaria* a été obtenu en reproduisant le procédé de l'exemple 1. On a réalisé en outre sur l'EXTRAIT 1, une étape supplémentaire de désodorisation par entraînement à la vapeur, de façon à améliorer les caractéristiques organoleptiques de l'extrait utilisé dans la composition cosmétique ci-dessous.

Une solution d'extrait a été utilisée à titre d'agent actif pour la préparation de la composition cosmétique ci-dessous (% exprimé en poids par rapport à la composition finale) :

| Phase A | |
|---|---|
| Phénoxyéthanol | 0,5 |
| Gomme xanthane | 0,2 |
| Acrylates/C20-30 alkylacrylate crospolymères | 0,2 |
| EDTA tétrasodique | 0,1 |
| Eau | qs |
| qs : quantité suffisante pour solubiliser les composés de la phase A. | |

| Phase B | |
|---|---|
| Extrait de *Kniphofia uvaria* selon l'invention | 1 |
| Polyisobutène hydrogéné | 4 |
| Squalane | 3 |
| Caprylique/caprique triglycérides | 3 |
| Pentylène glycol | 3 |
| Glycéryl stéarate | 3 |
| PEG-100 stéarate | 2,5 |
| Cire d'abeilles | 1,5 |
| Dicaprylyl carbonate | 1,5 |
| Cétyl alcool | 1 |
| Stéaryl alcool | 1 |
| Diméthicone | 1 |

| Phase C | |
|---|---|
| Hydroxyde de sodium | 0,04 |
| Eau | qs 100 |
| qs 100 : quantité suffisante pour 100% de la composition finale | |

On a dispersé les excipients de la phase A dans l'eau, puis on a chauffé à 80°C.

Les composés de la phase B, y compris l'extrait de *Kniphofia uvaria,* ont été chauffés à 85°C pour former une phase homogène. On a émulsionné la phase B dans la phase A à l'aide d'un mélangeur Ystral.

L'émulsion huile-dans-eau ainsi obtenue a été finalement neutralisée à l'aide d'une solution aqueuse d'hydroxyde de sodium 0,04 % p/p (phase C), puis refroidie.

La composition obtenue peut être utilisée comme crème destinée à être appliquée sur tout ou partie du visage.

Cette crème permet d'obtenir un effet de prévenir ou ralentir le vieillissement cutané, mais produit également un effet d'apaisement de la peau vis-à-vis d'espèces produisant un effet inflammatoire sur la peau.

On reproduit la formule en remplaçant l'EXTRAIT 1 par l'EXTRAIT 2 obtenu par pressage. La composition présente le même aspect que celle préparée ci-dessus.

## Revendications

1. Extrait des graines de la plante *Kniphofia uvaria* obtenu
- par pressage mécanique à froid desdites graines, ou
- par mise en contact desdites graines avec du dioxyde de carbone à l'état supercritique comme solvant, en absence de co-solvant, laquelle mise en contact est suivie de l'élimination dudit solvant.

2. Extrait selon la revendication 1, **caractérisé en ce que** le dioxyde de carbone à l'état supercritique a une température allant de 35°C à 80°C, et une pression supérieure à 7,4.10⁶ Pa.

3. Extrait selon la revendication 1, **caractérisé en ce que** le pressage mécanique est réalisé à froid sur les graines entières non préalablement broyées.

4. Composition cosmétique ou dermatologique comprenant un extrait tel que défini à l'une des revendications précédentes et au moins un excipient cosmétiquement ou dermatologiquement acceptable choisi parmi des pigments, des colorants, des polymères, des agents tensioactifs, des agents de rhéologie, des parfums, des ajusteurs de pH, des agents anti-oxydants, des conservateurs, et l'un quelconque de leurs mélanges.

5. Composition cosmétique ou dermatologique selon la revendication précédente, **caractérisé en ce qu'**elle comprend de 0,0001% à 10% en poids sec, d'extrait par rapport au poids total de la composition.

6. Utilisation dans une composition cosmétique d'au moins un extrait tel que défini à l'une des revendications 1 à 3,
- comme agent destiné à prévenir ou retarder l'apparition des signes du vieillissement cutané provoqué ou accéléré par les rayons UV, et/ou
- comme agent améliorant l'élasticité ou la fermeté de la peau, et/ou
- comme agent hydratant.

7. Composition dermatologique contenant au moins un extrait tel que défini à l'une des revendications 1 à 3, pour son utilisation :
- pour préserver la matrice extracellulaire d'une dégradation accélérée par les rayonnements UV,
- pour apaiser des peaux irritées sous l'effet des UV ou de l'utilisation d'alpha-hydroxyacides lors d'un gommage ou d'un « peeling ».

8. Composition dermatologique contenant au moins un extrait tel que défini à l'une des revendications 1 à 3, pour son utilisation dans le traitement du processus d'inflammation chronique dommageable pour la peau liée aux sécrétions des cellules stressées et/ou sénescentes, ou dans le traitement de la réactivité cutanée des peaux sensibles.

9. Méthode de soin cosmétique pour traiter les signes du vieillissement intrinsèque ou extrinsèque de la peau comprenant l'application d'une quantité efficace d'une composition telle que définie à l'une des revendications 4 ou 5 sur au moins une partie de la peau du visage ou du corps présentant des signes du vieillissement tels que des rides, un relâchement, une perte d'éclat du teint, une perte de fermeté et/ou d'élasticité de la peau, une diminution de l'épaisseur de la peau, une augmentation de la sécheresse ou une augmentation de la rugosité cutanée.

10. Méthode de soin cosmétique pour traiter les problèmes de sécheresse cutanée liée au vieillissement, qui mettent en jeu une baisse de la production de lipides qui contribue à la perturbation de la fonction barrière de la peau, comprenant l'application d'une quantité efficace d'une composition telle que définie à l'une des revendications 4 ou 5 sur au moins une partie de la peau du visage ou du corps présentant des signes du vieillissement tels que des rides, un relâchement, une perte d'éclat du teint, une perte de fermeté et/ou d'élasticité de la peau, une diminution de l'épaisseur de la peau, une augmentation de la sécheresse ou une augmentation de la rugosité cutanée.

## Patentansprüche

1. Extrakt aus den Samen der Pflanze *Kniphofia uvaria,* der erhalten wird
- durch mechanisches Kaltpressen der Samen oder
- durch Kontaktieren der Samen mit Kohlendioxid in superkritischem Zustand als Lösungsmittel, ohne Co-Lösungsmittel, an welches Kontaktieren sich das Entfernen des Lösungsmittels anschließt.

2. Extrakt nach Anspruch 1, **dadurch gekennzeichnet, dass** das Kohlendioxid in superkritischem Zustand eine Temperatur von 35 °C bis 80 °C und einen Druck von über 7,4.10⁶ Pa aufweist.

3. Extrakt nach Anspruch 1, **dadurch gekennzeichnet, dass** das mechanische Pressen an den ganzen, zuvor nicht gemahlenen Samen kalt durchgeführt wird.

4. Kosmetische oder dermatologische Zusammensetzung, umfassend einen Extrakt wie er in einem der vorhergehenden Ansprüche definiert ist sowie wenigstens einen kosmetisch oder dermatologisch akzeptablen Hilfsstoff, der aus Pigmenten, Farbstoffen, Polymeren, Tensiden, Rheologiemitteln, Duftstoffen, pH-Regulatoren, Antioxidantien, Konservierungsmitteln und einer ihrer Mischungen ausgewählt ist.

5. Kosmetische oder Dermatologische Zusammensetzung nach dem vorhergehenden Anspruch, **dadurch gekennzeichnet, dass** sie 0,0001 bis 10 Trockengewichtsprozent Extrakt bezogen auf das Gesamtgewicht der Zusammensetzung umfasst.

6. Verwendung, in einer kosmetischen Zusammensetzung, wenigstens eines Extraktes wie er in einem der Ansprüche 1 bis 3 definiert ist,
- als Wirkstoff, der dazu bestimmt ist, dem Auftreten der Zeichen der durch UV-Strahlen bewirkten oder beschleunigten Hautalterung vorzubeugen oder es zu verzögern, und/oder
- als Wirkstoff, der die Elastizität oder die Festigkeit der Haut verbessert, und/oder
- als hydratisierender Wirkstoff.

7. Dermatologische Zusammensetzung, die wenigstens einen Extrakt, wie er in einem der Ansprüche 1 bis 3 definiert ist, enthält, für ihre Verwendung:
- um die extrazelluläre Matrix vor einem beschleunigten Abbau durch die UV-Strahlungen zu schützen,
- um irritierte Haut unter der Wirkung der UV-Strahlen oder der Verwendung von Alpha-Hydroxysäuren während einer Schälkur oder eines "Peeling" zu beruhigen.

8. Dermatologische Zusammensetzung, die wenigstens einen Extrakt, wie er in einem der Ansprüche 1 bis 3 definiert ist, enthält, für ihre Verwendung bei der Behandlung des Prozesses einer für die Haut schädlichen chronischen Entzündung, verbunden mit Sekretionen der gestressten und/oder gealterten Zellen, oder bei der Behandlung der Hautreaktivität empfindlicher Haut.

9. Kosmetisches Pflegeverfahren zur Behandlung der Zeichen der intrinsischen oder extrinsischen Alterung der Haut, umfassend das Auftragen einer wirkungsvollen Menge einer Zusammensetzung, wie sie in einem der Ansprüche 4 oder 5 definiert ist, auf wenigstens einen Teil der Haut des Gesichts oder des Körpers, der Zeichen der Alterung, wie Falten, ein Erschlaffen, einen Glanzverlust des Teints, einen Verlust von Festigkeit und/oder Elastizität der Haut, eine Verringerung der Dicke der Haut, eine Erhöhung der Trockenheit oder eine Erhöhung der Hautrauheit aufweist.

10. Kosmetisches Pflegeverfahren zur Behandlung der Probleme einer mit der Alterung verbundenen Hauttrockenheit, die eine Abnahme der Lipidproduktion, welche zur Störung der Barrierefunktion der Haut beiträgt, ins Spiel bringen, umfassend das Auftragen einer wirkungsvollen Menge einer Zusammensetzung, wie sie in einem der Ansprüche 4 oder 5 definiert ist, auf wenigstens einen Teil der Haut des Gesichts oder des Körpers, der Zeichen der Alterung, wie Falten, ein Erschlaffen, einen Glanzverlust des Teints, einen Verlust von Festigkeit und/oder Elastizität der Haut, eine Verringerung der Dicke der Haut, eine Erhöhung der Trockenheit oder eine Erhöhung der Hautrauheit aufweist.

## Claims

1. An extract of the seeds of Kniphofia uvaria plant, obtained
- by mechanical pressing of said seeds, under cold conditions, or
- by bringing said seeds into contact with carbon dioxide in a supercritical state as solvent, in the absence of co-solvent, and then removing said solvent.

2. The extract as claimed in claim 1, **characterized in that** the carbon dioxide in a supercritical state has a temperature ranging from 35°C to 80°C, and a pressure above 7.4 × 10⁶ Pa.

3. The extract as claimed in claim 1, **characterized in that** the mechanical pressing is carried out under cold conditions on whole seeds which have not been preground in a previous step.

4. A cosmetic or dermatological composition comprising an extract as defined in any one of the preceding claims and at least one cosmetically or dermatologically acceptable excipient chosen from pigments, dyes, polymers, surfactants, rheology agents, fragrances, pH adjusters, antioxidants, preservatives, and any mixture thereof.

5. The cosmetic or dermatological composition as claimed in the preceding claim, **characterized in that** it comprises from 0.0001% to 10% by dry weight, of extract to the total weight of the composition.

6. Use of at least one extract as defined in claims 1 to 3 in a cosmetic composition,
- as an agent intended for preventing or delaying the appearance of the signs of skin aging caused or accelerated by UV rays, and/or
- as an agent for improving the elasticity or the firmness of the skin, and/or
- as a moisturizing agent.

7. A dermatological composition containing at least one extract as defined in claims 1 to 3, for its use:
- for preserving the extracellular matrix against degradation accelerated by UV radiation,
- for soothing skin which is irritated under the effect of UV radiation or by the use of alpha-hydroxy acids during exfoliation or a peel.

8. A dermatological composition containing at least one extract as defined in claims 1 to 3, for its use in the treatment of the process of chronic inflammation harmful to the skin associated with secretions by stressed and/or senescent cells, or in the treatment of cutaneous reactivity of sensitive skin.

9. A cosmetic care method for treating the signs of intrinsic or extrinsic aging of the skin comprising the step of applying an effective amount of a composition as defined in any one of claim 4 or 5 to at least one part of the skin of the face or the body exhibiting visible signs of aging such as wrinkles, a slackening, a loss of radiance of the complexion, a loss of firmness and/or of elasticity of the skin, a decrease in the thickness of the skin, an increase in the dryness or in the roughness of the skin.

10. A cosmetic care method for treating problems of dryness of the skin associated with aging, which involve a reduction in the production of lipids which contributes to the disruption of the barrier function of the skin, comprising the step of applying an effective amount of a composition as defined in any one of claim 4 or 5 to at least one part of the skin of the face or the body exhibiting visible signs of aging such as wrinkles, a slackening, a loss of radiance of the complexion, a loss of firmness and/or of elasticity of the skin, a decrease in the thickness of the skin, an increase in the dryness or in the roughness of the skin.
